# EUROPÄISCHE PATENTSCHRIFT

(11) **EP 1 601 686 B1**
(45) Veröffentlichungstag und Bekanntmachung des Hinweises auf die Patenterteilung: **14.07.2010**
(21) Anmeldenummer: 04717089.9
(22) Anmeldetag: 04.03.2004
(51) Int. Cl.: C07K 9/00, C07H 15/252, A61K 38/08, A61P 35/00

(54) **PROTEIN-BINDENDE DOXORUBICIN-PEPTID-DERIVATE**
PROTEIN-BINDING DOXORUBICIN PEPTIDE DERIVATIVES
DERIVES DE PEPTIDES DE LA DOXORUBICINE LIANT DES PROTEINES

(30) Priorität: 07.03.2003 DE 10310082
(43) Veröffentlichungstag der Anmeldung: 07.12.2005
(73) Patentinhaber: KTB Tumorforschungs GmbH, 79106 Freiburg (DE)
(72) Erfinder: KRATZ, Felix, 79241 Ihringen (DE)
(74) Vertreter: Dey, Michael
(86) Internationale Anmeldenummer: PCT/EP2004/002204
(87) Internationale Veröffentlichungsnummer: WO 2004/078781

(56) Entgegenhaltungen:
- DE-A- 19 926 154
- KRATZ F ET AL: "Development and in vitro efficacy of novel MMP2 and MMP9 specific doxorubicin albumin conjugates" 6. August 2001 (2001-08-06), BIOORGANIC & MEDICINAL CHEMISTRY LETTERS, OXFORD, GB, PAGE(S) 2001-2006 , XP002208108 ISSN: 0960-894X Verbindungen 1c und 2c das ganze Dokument

## Beschreibung

Die Erfindung betrifft Protein-bindende Doxorubicin-Peptid-Derivate, die durch die Matrix-Metalloproteinasen 2 bzw. 9 spaltbar sind, deren Herstellung und Verwendung.

Die chemotherapeutische Behandlung maligner Erkrankungen mit Doxorubicin ist aufgrund einer geringen therapeutischen Breite dieses Wirkstoffes mit Nebenwirkungen verbunden (Dorr RT, Von Hoff DD, "Cancer Chemotherapy Handbook", 2nd ed. Appleton and Lange, Norwalk, 1994). Es ist deshalb für eine effektivere Behandlung wünschenswert, die systemische Toxizität dieses Zytostatikums zu reduzieren und gleichzeitig dessen pharmakologisches Potenzial zu steigern. Es ist bekannt, dass mit bestimmten Prodrugs einerseits ein gezielter Transport von gebundenen Wirkstoffen ins befallene Gewebe und andererseits ein effizientes und möglichst spezifisches Freisetzen des Wirkstoffs am Zielort infolge biochemischer oder physiologischer Besonderheiten des malignen Gewebes erreicht werden konnte. Ein Ansatz zur Verbesserung des Nebenwirkungsprofils und der Wirksamkeit von Zytostatika liegt in der Entwicklung Proteinbindender Formulierungen, welche *in vivo* an endogene Serumproteine, insbesondere Albumin, koppeln und auf diese Weise makromolekulare Transportformen der Wirksstoffe darstellen (Kratz, F. et al., J. Med Chem. 2000, 43, 1253-1256).

Kratz et al. (Bioorg. Med. Chem. Lett. 11 (2001), 2001-2006) beschreiben zwei Doxorubicin-Albumin-Konjugate (A-DP1 und A-DP2), welche sich in ihrer Substratspezifität für die Matrix-Metallproteinasen MMP-2 und MMP-9 unterscheiden und durch Bindung von Maleimid-Doxorubicin-Peptidderivaten an die Cystein-34-Position von Humanserumalbumin hergestellt werden. DE 199 26 154 offenbart eine injizierbare Arzneimittelzubereitung enthaltend eine pharmakologisch aktive Substanz, die in einer injizierbaren Trägerflüssigkeit gelöst wird und aus einem Wirkstoff und wenigstens einem Protein-bindenden Molekülrest besteht, die durch einen Spacer verbunden sind, wobei der Spacer oder die Bindung zwischen dem Wirkstoff in dem Spacer pH-abhängig oder enzymatisch im Körper spaltbar ist unter Freisetzung des Wirkstoffs.

Weiterhin sind Matrix-Metalloproteinasen (MMP), insbesondere MMP-2 und MMP-9, als wichtige Proteasen bei der Progression von bösartigen Tumoren identifiziert worden (Stetler-Stevenson, W.G. et al., Annu. Rev. Cell Biol. 1993, 9, 541-573).

Der Erfindung liegt die Aufgabe zugrunde zur Vergrößerung der therapeutischen Breite von Doxorubicin, Derivate dieses Wirkstoffes zu schaffen, die nach intravenöser Applikation kovalent an zirkulierendes Albumin binden und durch MMP-2 bzw. MMP-9 im Tumorgewebe gespalten werden. Diese Aufgabe wird erfindungsgemäß gelöst durch niedermolekulare Doxorubicin-Peptid-Derivate der allgemeinen Formel I 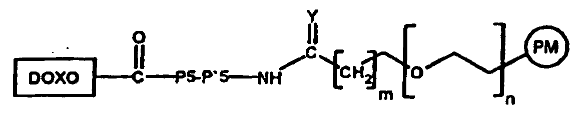 in der 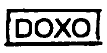 = Doxorubicin (Formel nachstehend)
Y = O oder S
n = 1 1 bis 5
m = 0 bis 6
P₅-P'₅ eine Peptidsequenz bestehend aus bis zu zehn der 20 essenziellen Aminosäuren bedeuten und PM eine Protein-bindende Gruppe ist, 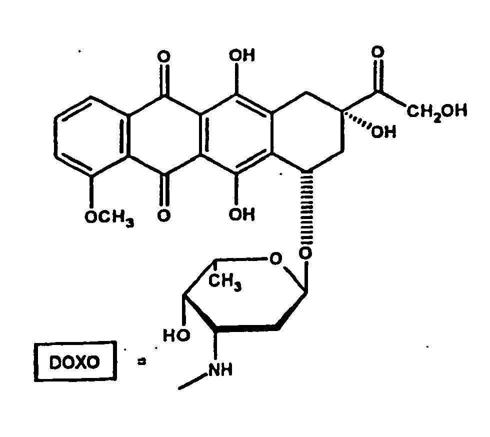 wobei die Protein-bindende Gruppe (PM) ausgewählt ist unter einer 2-Dithiopyridylgruppe, einer Halogenacetamidgruppe, einer Halogenacetatgruppe, einer Disulfidgruppe, einer Acylsäureestergruppe, einer Monoalkylmaleinsäureestergruppe, einer Monoalkylmaleaminsäureamidgruppe, einer N-Hydroxysuccinimidylestergruppe, einer Isothiocyanatgruppe, einer Aziridingruppe oder einer Maleinimidgruppe.

Die erfindungsgemäßen Verbindungen sind aus Doxorubicin, einem Peptidspacer und einem heterobifunktionellen Crosslinker, enthaltend eine Protein-bindende Gruppe PM aufgebaut. Im Folgenden wird dieser Aufbau näher erläutert:

Der heterobifunktionelle Crosslinker ist ein Carbonsäure-Derivat mit einer Protein-bindenden Gruppe der allgemeinen Formel II 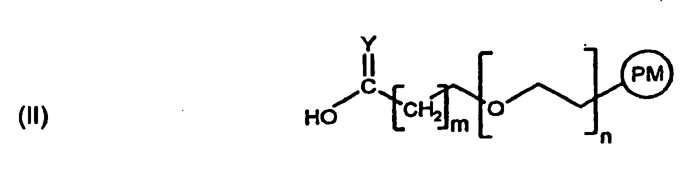 in der
Y = O oder S
n = 1 bis 5
m = 0 bis 6
PM = Protein-bindende Gruppe
bedeuten.

Bevorzugt eingesetzt werden heterobifunktionelle Crosslinker mit m < 2 und n = 2 bis 5. Die Oxyethylen-Einheiten gewährleisten eine erhöhte Wasserlöslichkeit, insbesondere bei den größeren Werten von n.

Eine besonders bevorzugte Protein-bindende Gruppe ist die Maleinimidgruppe.

Der Peptidspacer ist eine Peptidsequenz P₅-P'₅, bestehend aus bis zu zehn der 20 essenziellen Aminosäuren, die durch MMP-2 bzw. MMP-9 spaltbar ist. Bevorzugte Peptidspacer bestehen aus acht Aminosäuren. Besonders bevorzugte Sequenzen sind:

| Peptid | | | | | | | | | |
|---|---|---|---|---|---|---|---|---|---|
| P₅ | P₄ | P₃ | P₂ | P₁ | | P'₁ P'₂ | P'₃ | P'₄ | P'₃ |
| Gly-Pro-Gln-Gly-Ile-Ala-Gly-Gln | | | | | | | | | |
| Gly-Pro-Leu-Gly-Ile-Ala-Gly-Gln | | | | | | | | | |
| Gly-Pro-Gln-Gly-Ile-Trp-Gly-Gln | | | | | | | | | |

Diese Peptide werden durch MMP-2 bzw. MMP-9 an der P₁-P'₁-Bindung enzymatisch gespalten.

Am meisten bevorzugt ist die Sequenz Gly-Pro-Leu-Gly-Ile-Ala-Gly-Gln.

Die Herstellung der erfindungsgemäßen Doxorubicin-Peptid-Derivate erfolgt zweckmäßig durch Umsetzung von Doxorubicin mit einem Peptid-Derivat der allgemeinen Formel III 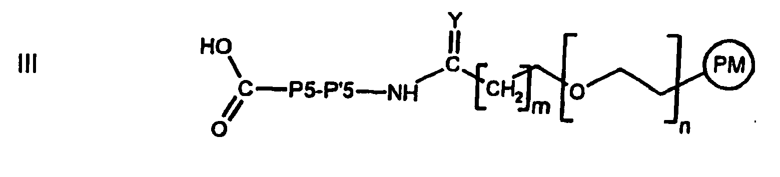 in der
Y = O oder S
n = 1 bis 5
m = 0 bis 6
P₅-P'₅ eine Peptidsequenz, bestehend aus bis zu zehn der 20 essenziellen Aminosäuren und PM eine Protein-bindende Gruppe bedeuten, durch Kondensation der aktivierten Carboxylgruppe des Peptid-Derivats mit der Aminogruppe des Daunosaminrings von Doxorubicin.

Als Reagenzien zur Aktivierung des C-terminalen Endes des Peptid-Derivats werden vorzugsweise N,N'-Dicyclohexylcarbodiimid, N,N'-Diisopropylcarbodiimid, (Benzotriazol-1-yloxy)tris(dimethylamino)phosphonium-hexafluorophosphat oder 2-Chlor-1-methyl-pyrdiniumiodid unter Zusatz gängiger Katalysatoren bzw. Hilfsbasen wie z.B. Trialkylamine, Pyridin, 4-Dimethylaminopyridin (DMAP) oder Hydroxybenzotriazol (HOBt) eingesetzt. Die Umsetzungen werden zweckmäßig in polaren Lösemitteln, etwa DMF, DMA bzw. DMSO, bei Temperaturen zwischen -20 °C und 40 °C, bevorzugt bei 0 bis 5 °C, durchgeführt, wobei die Reaktionszeit normalerweise zwischen 1 und 120 h, bevorzugt zwischen 24 und 96 h, liegt. Die Produktisolierung kann durch übliche Methoden, wie Kristallisation, Chromatographie an Kieselgel oder Chromatographie, erfolgen.

Die Herstellung des Peptid-Derivats erfolgt vorzugsweise durch Umsetzung der aktivierten Carboxylgruppe des heterobifunktionellen Crosslinkers der allgemeinen Formel II mit dem IV-terminalen Ende der Peptidsequenz P₅-P'₅. Dabei werden als Reagenzien zur Aktivierung der Carboxylgruppe des Crosslinkers vorzugsweise N,N'-Dicyclohexylcarbodiimid, N,N'-Diisopropylcarbodiimid, (Benzotriazol-1-yloxy)-tris(dimethylamino)phosphonium-hexafluorophosphat oder 2-Chlor-1-methylpyridiniumiodid unter Zusatz gängiger Katalysatoren bzw. Hilfsbasen wie z.B. Trialkylamine, Pyridin, 4-Dimethylaminopyridin (DMAP) oder Hydroxybenzotriazol (HOBt) eingesetzt. Die Umsetzung erfolgt zweckmäßig an der Festphase, und die Produktisolierung erfolgt in der Regel durch Reversed-Phase-Chromatographie (präparative HPLC), wie dem Fachmann geläufig ist.

Die erfindungsgemäßen Protein-bindenden Doxorubicin-Peptid-Derivate werden parenteral, bevorzugt intravenös appliziert. Dazu werden die erfindungsgemäßen Doxorubicin-Peptid-Derivate als Lösungen, Feststoffe oder Lyophilisate, gegebenenfalls unter Verwendung üblicher Hilfsstoffe bereitgestellt. Solche Hilfsstoffe sind beispielsweise Polysorbate, Glucose, Lactose, Mannitol, Saccharose, Dextrane, Zitronensäure, Tromethamol, Triethanolamin, Aminoessigsäure und/oder synthetische Polymere. Bevorzugt werden die erfindungsgemäßen Doxorubicin-Peptid-Derivate in einem isotonischen Puffer in einem pH-Bereich von 2,0-8,0, bevorzugt pH 5,0 bis 7,0, gelöst und appliziert. In der Regel besitzen die erfindungsgemäßen Doxorubicin-Peptid-Derivate eine ausreichende Wasserlöslichkeit aufgrund der Oxyethylen-Einheiten im Crosslinker und/oder der Integration von polaren Aminosäuren in die Peptidsequenz, wie z.B. Arginin, Prolin, Glutamin und/oder Glutaminsäure. Die Löslichkeit des Doxorubicin-Peptid-Derivates kann gegebenenfalls durch pharmazeutische Lösungsmittel wie etwa 1,2-Propandiol, Ethanol, Isopropanol, Glycerol und/oder Poly(ethylenglykol) mit einem Molekulargewicht von 200 bis 600 g/mool, bevorzugt Poly(ethylenglykol) mit einem Molekulargewicht von 600 g/mol und/oder Löslichkeitsvermittler wie z.B. Tween 80, Cremophor oder Polyvinylpyrrolidon verbessert werden.

Ein wesentliches Merkmal der erfindungsgemäßen Doxorubicin-Peptid-Derivate liegt in einer raschen kovalenten Bindung an Serumproteine über die Protein-bindende Gruppe, wodurch eine makromolekulare Transportform des Wirkstoffs generiert wird. Von Serumproteinen wie Transferrin oder Albumin, ist eine erhöhte Aufnahme in Tumorgewebe bekannt (Kratz F.; Beyer U. Drug Delivery 1998, 5, 281-299), sodass diese im Rahmen der Erfindung als endogene Träger für Zytostatika herangezogen werden können. Ein besonders bevorzugtes Serumprotein ist zirkulierendes Humanserumalbumin (HSA), das mit einer durchschnittlichen Konzentration von 30 bis 50 g/l die Hauptprotein-Komponente des menschlichen Blutes bildet (Peters T. Adv. Protein Chem. 1985, 37, 161-245) und eine freie Cysteingruppe (Cystein-34-Gruppe) an der Oberfläche des Proteins aufweist, welche zur Anbindung von Thiol-bildenden Gruppen wie Maleinimiden oder Disulfiden geeignet ist (WO 00/76551). Die Reaktion der Doxorubicin-Peptid-Derivate mit Serumproteinen kann auch extrakorporal durchgeführt werden, z.B. mit einer zur Infusion vorgesehenen Albumin-, Blut- oder Serummenge.

Protein-gebundene Doxorubicin-Peptid-Derivate weisen gegenüber Doxorubicin eine veränderte Bioverteilung auf und reichern sich aufgrund ihres makromolekularen Charakters in Tumorgewebe an. Durch eine dort stattfindende Spaltung durch MMP-2 bzw. MMP-9 werden niedermolekulare Doxorubicinpeptide abgespalten, welche im Tumorgewebe Doxorubicin als aktive Komponente freisetzen. In Tierversuchsstudien zeigten Protein-bindende Doxorubicin-Peptid-Derivate überraschend auch eine höhere Wirksamkeit als der klinische Standard Doxorubicin (siehe Beispiel 1).

Die folgenden Beispiele erläutern die Erfindung in Verbindung mit der Zeichnung näher. In der Zeichnung stellen dar:
Figur 1 die Strukturformel des Produkts von Beispiel 1;
Figur 2 ein Chromatogramm eines Produkts der Inkubation der Verbindung von Figur 1 mit Humanplasma;
Figur 3 ein Chromatogramm eines Spaltungsansatzes der Albumingebundenen Form der Verbindung von Figur 1 durch N4MP-2;
Figur 4 ein Chromatogramm eines Inkubationsansatzes von gespaltenem Doxorubicintetrapeptid mit A375-Melanoma-Gewebehomogenat;
Figur 5 eine graphische Darstellung des Tumorwachstums von A375 Melanom mit Doxorubicin einerseits und der Verbindung von Figur 1 andererseits.

### Beispiel 1

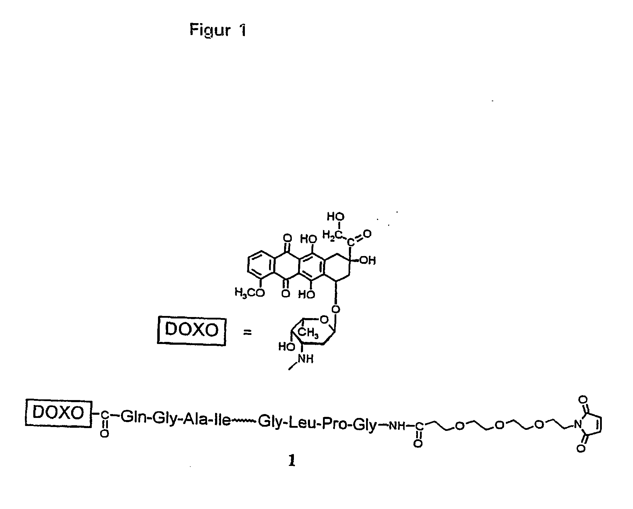

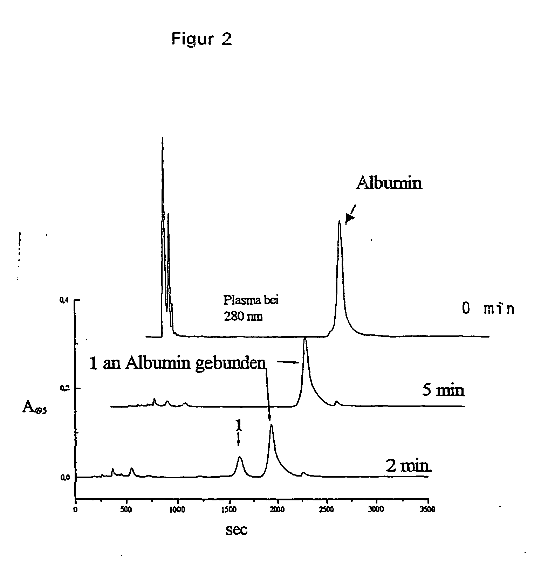

**Synthese von 1 (siehe** **Figur 1****):** 175,0 mg (0,3 mmol) Doxorubicin-Hydrochlorid, 298,5 mg (0,3 mmol) Mal-Gly-Pro-Leu-Gly-Ile-Ala-Gly-Gln (Mal = Maleinimidotriethylenglykolsäure), 40,5 mg (0,3 mmol) 1-Hydroxybenzotriazol-Hydrat und 98,95 µl (91,0 mg, 0,9 mmol) 4-Methylmorpholin wurden in 50 ml wasserfreiem N,N-Dimethylformamid (DMF) bei + 5 °C 15 Minuten lang gerührt. 139,36 µl (113,6 mg, 0,9 mmol) N,N'-Diisopropylcarbodiimid wurden zugegeben und der Ansatz bei + 5 °C 72 Stunden lang gerührt. Anschließend wurde das Lösungsmittel im Hochvakuum entfernt, der Rückstand in einer Minimalmenge Chloroform/Methanol 4:1 gelöst und das Produkt durch zweimalige Säulenchromatographie an Kieselgel 60 (Merck, Darmstadt) mit Chloroform/Methanol 4:1 aufgereinigt. Aus den erhaltenen Fraktionen wurde **1** durch Zugabe eines Überschusses an Diethylether gefällt und abzentrifugiert, mit 2 x 20 ml Diethylether gewaschen und erneut zentrifugiert. Nach Trocknen im Hochvakuum wurden 250 mg 1 als rostes Pulver erhalten. Masse (MALDI-TOF, Mr 1520.7): *m*/*z* 1543 [M + Na]⁺, HPLC (495 nm): > 98 %. 1 enthält Maleinimidotriethylenglykolsäure als wasserlösliche und Albuminbindende Komponente. 1 bindet innerhalb weniger Minuten selektiv an die Cystein-34-Position von endogenem Albumin im Blutplasma (siehe Figur 2).

Figur 2 zeigt Chromatogramme einer Inkubationsstudie von 1 mit Humanplasma bei 37 °C nach 2 und 5 min. Konzentration von 1 = 59 µM. HPLC: BioLogic Duo-Flow System von Biorad, München; Lambda 1000 Monitor von Bischoff (λ = 495 nm) und Merck F-1050 Fluoreszenz-Spektrophotometer (EX. 490 nm, EM. 540 nm); UV-Detektion bei 280 nm; Säule: Waters, 300 Å, Symmetry C18 [4,6 x 250 mm] mit Vorsäule; Fluss: 1,2 ml/min, mobile Phase A: 27,5 % CH₃CN, 72,5 % 20 mM Kaliumphosphat (pH 7,0), mobile Phase B: CH₃CN, Gradient: 0-25 min 100 % mobile Phase A; in 25 bis 40 min auf 70 % CH₃CN, 30 % 20 mM Kaliumphosphat; 40 bis 50 min 70 % CH₃CN, 30 % 20 mM Kaliumphosphat; 50-60 min 100 % mobile Phase A; Injektionsvolumen: 50 µl.

Die Chromatogramme zeigen, dass nach 2 min Inkubation bereits ein erheblicher Teil von **1** an Albumin gebunden vorliegt; nach 5 min ist die gesamte Menge **1** an Albumin gebunden.

### Beispiel 2

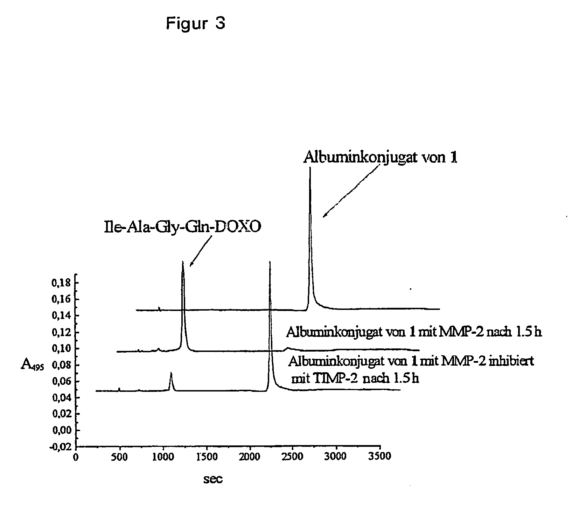

Die Albumin-gebundene Form von **1** wurde 1,5 Stunden mit 2 mU aktivierter MMP-2 bei 37 °C in Abwesenheit und Anwesenheit eines zweifachen Überschusses an TIMP-2 (tissue inhibitor of MMP-2) inkubiert. Der Spaltungsansatz wurde dann einer HPLC unter den Bedingungen wie zu Figur 2 unterzogen. Die Konzentration an Anthracyklin war 100 µm. Das Chromatogramm wird in Figur 3 dargestellt. Ähnliche Ergebnisse wurden bei Verwendung von MMP-9 anstelle von MMP-2 erzielt (MMP-2 und MMP-9 von Calbiochem FRG). Das Chromatogramm des Ansatzes mit MMP-2 und ohne TIMP-2 zeigt, dass nach 1,5 Stunden aus dem Albuminkonjugat von 1 vollständig der Rest Ile-Ala-Gly-Gln-DOXO abgespalten war. Bei Hemmung von MMP-2 durch TIMP-2 war nur eine sehr geringe Spaltung erfolgt und das Albuminkonjugat von **1** fast vollständig erhalten geblieben.

### Beispiel 3

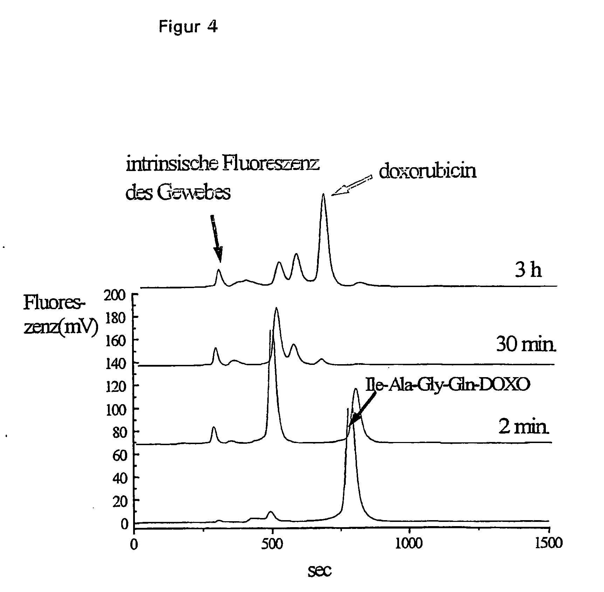

Mit dem wie in Beispiel 2 beschrieben, erhaltenen gespaltenten Doxorubicintetrapeptid (Ile-Ala-Gly-Gln-DOOXO) wurde eine Inkubationsstudie mit A375-Melanome-Gewebehomogenat durchgeführt. Die Inkubation erfolgte bei 37 °C. Die Konzentration an Anthracyklin war 100 µm. Nach 2 min, 30 min und 3 Stunden wurde jeweils eine HPLC-Chromatographie unter den Bedingungen von Figur 2 durchgeführt. Das für diese Versuche verwendete Melanoma-Gewebehomogenat wurde mit A375 Xenograft-Tumoren in 50 mM Tris-HCL-Puffer pH 7,4, enthaltend 1 mM Monothioglycerol hergestellt. Figur 4 zeigt die dabei erhaltenen Ergebnisse.

### Beispiel 4

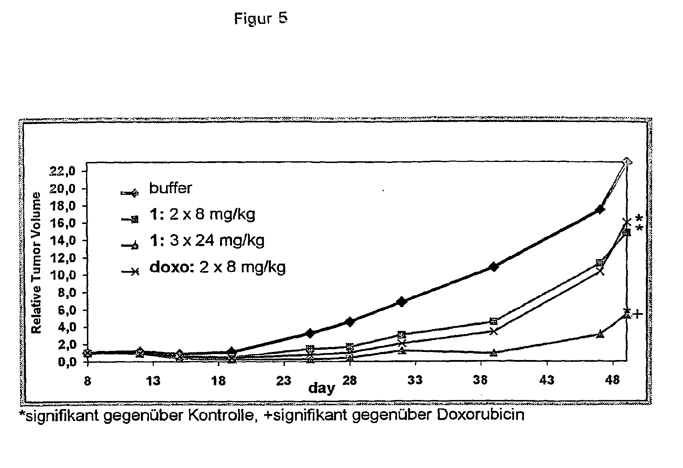

Der Verlauf des Tumorwachstums von subkutan wachsenden A375 Melanoma-Xenografts, die mit Doxorubicin und **1** [Dosis (i.v.); Doxorubicin (= doxo): 2 x 13,3 µmol/kg = 2 x 8 mg/kg Doxorubicin) an den Tagen 8 und 15; 1: 2 x 13,3 µmol/kg (= 2 x 8 mg/kg Doxorubicin-Äquivalente) an den Tagen 8 und 15, 3 x 39,9 µmol/kg ( = 3 x 24 mg/kg Doxorubicinäquivalente) an den Tagen 8, 15 und 22 behandelt wurden, wird in Figur 5 gezeigt. Dargestellt ist das relative Tumorvolumen zu den angegebenen Zeiten. Tiere: Nacktmäuse; Stammlösung von Doxorubicin (2 mg/ml); Stammlösung von **1**: 6 mg/ml in 10 mM Natriumphosphat, 5 % D-Glucose (pH 6,4), Kontrolle (Puffer): Glucose-Phosphat-Puffer (10 mM Natriumphosphat, 5 % D-Glucose - pH 6,4) an den Tagen 8 und 15.

Die Kurven in Figur 5 zeigen ganz klar die große Überlegenheit des erfindungsgemäßen Doxorubicinderivates im Vergleich zu Dozorubicin. Doxorubicin ergibt dabei ein etwa dreimal so großes Tumorvolumen vergleichen mit der Behandlung mit dem erfindungsgemäßen Derivat. Dies zeigt die überraschend bessere Wirksamkeit, die mit dem erfindungsgemäßen Derivat erzielt wird.

## Patentansprüche

1. Doxorubicin-Peptid-Derivat der allgemeinen Formel I: 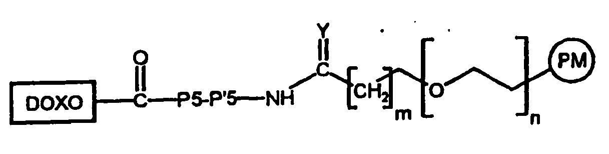 in der
Y = O oder S
n = 1 bis 5
m = 0 bis 6
P₅-P'₅ eine durch Matrix-Metalloproteinase 2 bzw. 9 (MMP-2 bzw. MMP-9) spaltbare Peptidsequenz, bestehend aus bis zu zehn der zwanzig essenziellen Aminosäuren, bedeuten und PM eine Protein-bindende Gruppe ist und 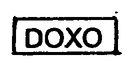 = Doxorubicin der Formel ist 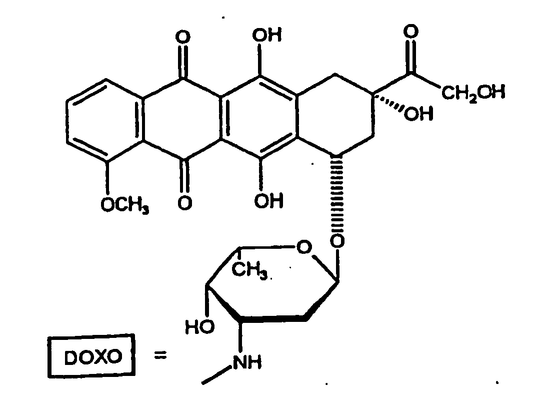 wobei PM ausgewählt ist unter einer Maleinimidgruppe, einer 2-Dithiopyridylgruppe, einer Halogenacetamidgruppe, einer Halogenacetatgruppe, einer Disulfidgruppe, einer Acrylsäureestergruppe, einer Monoalkylmaleinsäureestergruppe, einer Monoalkylmaleaminsäureamidgruppe, einer N-Hydroxysuccinimidylestergruppe, einer Isothiocyanatgruppe oder einer Aziridingruppe, die gegebenenfalls substituiert sein können.

2. Doxorubicin-Peptid-Derivat nach Anspruch 1, wobei PM eine Maleinimidgruppe ist, die gegebenenfalls substituiert sein kann.

3. Doxorubicin-Peptid-Derivat nach Anspruch 1 oder 2,
**dadurch gekennzeichnet,**
**dass** m < 2 ist.

4. Doxorubicin-Peptid-Derivat nach einem der Ansprüche 1 bis 3,
**dadurch gekennzeichnet,**
**dass** Y = O ist.

5. Doxorubicin-Peptid-Derivat nach einem der vorhergehenden Ansprüche,
**dadurch gekennzeichnet,**
**dass** die Peptidsequenz P₅-P'₅ acht Aminosäuren umfasst.

6. Doxorubicin-Peptid-Derivat nach Anspruch 5,
**dadurch gekennzeichnet,**
**dass** die Peptidsequenz Gty-Pro-Gln-Gly-Ile-Ala-Gly-Gln, Gly-Pro-Leu-Gly-Ile-Ala-Gly-Gln oder Gly-Pro-Gln-Gly-Ile-Trp-Gly-Gln ist.

7. Doxorubicin-Peptid-Derivat nach Anspruch 5,
**dadurch gekennzeichnet,**
**dass** die Peptidsequenz Gly-Pro-Leu-Gly-Ile-Ala-Gly-Gln ist.

8. Verfahren zur Herstellung von Doxorubicin-Peptid-Derivaten nach einem der vorhergehenden Ansprüche,
**dadurch gekennzeichnet, dass**, mit einem Peptid-Derivat der allgemeinen Formel II
Doxorubicin 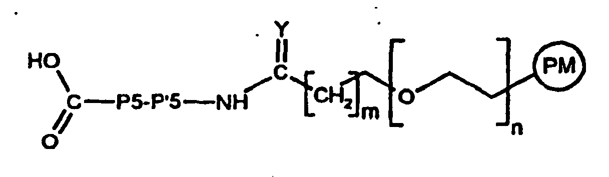 in der
Y =O oder S
n = 1 bis 5
m = 0 bis 6
P₅-P'₅ eine Peptidsequenz, bestehend aus bis zu zehn der zwanzig essenziellen Aminosäuren, und PM eine Protein-bindende Gruppe bedeuten
in Gegenwart von Carbonsäure-Aktivierungsreagenz umgesetzt wird.

9. Verfahren nach Anspruch 8,
**dadurch gekennzeichnet,**
**dass** PM eine Maleinimidgruppe ist.

10. Verfahren nach Anspruch 8 oder 9,
**dadurch gekennzeichnet,**
**dass** das Peptid-Derivat der allgemeinen Formel II durch Umsetzung einer Peptidsequenz P₅-P'₅ mit einem heterobifunktionellen Crosslinker der allgemeinen Formel III 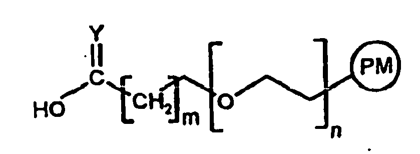 in der
Y = O oder S
n = 1 bis 5
m = 0 bis 6
PM = Protein-bindende Gruppe bedeuten
in Gegenwart von Carbonsäure-Aktivierungsreagenz erhalten wird.

11. Verfahren nach einem der Ansprüche 8 bis 10,
**dadurch gekennzeichnet,**
**dass** das Carbonsäure-Aktivierungsreagenz unter N,N'-Dicyclohexylcarbodiimid, N,N'-Diisopropylcarbodiimid oder (Benzotriazol-1-yloxy)-tris(dimethylamio)phosphonium-Hexafluorphosphat, bevorzugt N,N'-Diisopropylcarbodiimid ausgewählt wird.

12. Arzneimittel,
**gekennzeichnet durch**
einen Gehalt an Doxorubicin-Peptid-Derivat gemäß einem der Ansprüche 1 bis 8 als Wirkstoff, gegebenenfalls zusammen mit üblichen pharmazeutischen Hilfsstoffen und/oder Lösungsmitteln.

13. Verwendung eines Doxorubicin-Peptid-Derivats nach einem der Ansprüche 1 bis 7 zur Herstellung eines Arzneimittels zur Behandlung von Krebskrankheiten.

14. Verfahren zur Herstellung eines Arzneimittels für die Behandlung von Krebskrankheiten,
**dadurch gekennzeichnet,**
**dass** man eine Verbindung gemäß einem der Ansprüche 1 bis 7 in eine therapeutisch annehmbare Lösung überführt.

## Claims

1. Doxorubicin-peptide derivative of the general formula I 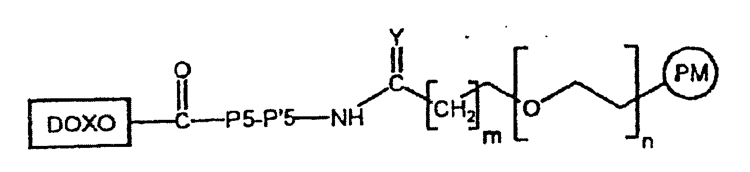 in which
Y = O or S
n = 1 to 5
m = 0 to 6
P₅-P'₅ denotes a peptide sequence consisting of up to ten of the twenty essential amino acids that can be cleaved by the matrix metalloproteinase 2 and/or 9 (MMP-2 and/or MMP-9) and PM is a protein-binding group and [doxo] = doxorubicin is of the formula 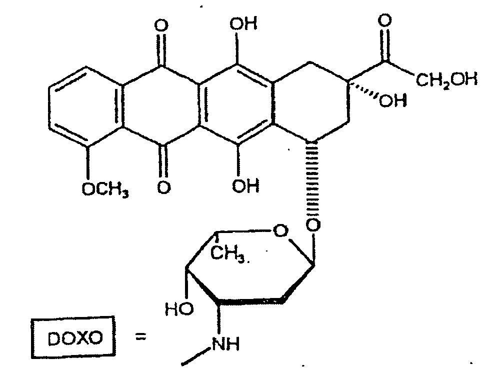 where PM is selected from a maleinimide group, a 2-dithiopyridyl group, a halogen acetamide group, a halogen acetate group, a disulfide group, an acrylic acid ester group, a monoalkylmaleic acid ester group, a monoalkylmaleamic acid amide group, an N-hydroxysuccinimidyl ester group, an isothiocyanate group or an aziridine group which can be optionally substituted.

2. Doxorubicin-peptide derivative according to claim 1, wherein PM is a maleinimide group which can be optionally substituted.

3. Doxorubicin-peptide derivative according to claim 1 or 2, **characterized in that**
m is < 2.

4. Doxorubicin-peptide derivative according to one of the claims 1 to 3, **characterized in that**
Y=O.

5. Doxorubicin-peptide derivative according to one of the previous claims , **characterized in that**
the peptide sequence P₅-P'₅ comprises eight amino acids.

6. Doxorubicin-peptide derivative according to claim 5,
**characterized in that**
the peptide sequence is Gly-Pro-Gln-Gly-Ile-Ala-Gly-Gln, Gly-Pro-Leu-Gly-Ile-Ala-Gly-Gln or Gly-Pro-Gln-Gly-Ile-Trp-Gly-Gln.

7. Doxorubicin-peptide derivative according to claim 5,
**characterized in that**
the peptide sequence is Gly-Pro-Leu-Gly-Ile-Ala-Gly-Gln.

8. Process for producing doxorubicin-peptide derivatives according to one of the previous claims,
**characterized in that**
doxorubicin is reacted with a peptide derivative of the general formula II 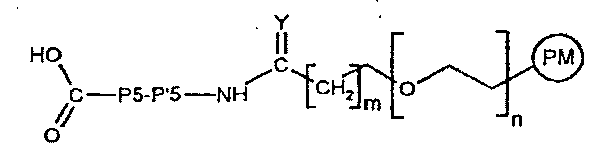 in which
Y = O or S
n = 1 to 5
m = 0 to 6
P₅-P'₅ denotes a peptide sequence consisting of up to ten of the twenty essential amino acids and PM denotes a protein-binding group,
in the presence of a carboxylic acid activation reagent.

9. Process according to claim 8,
**characterized in that**
PM is a maleinimide group.

10. Process according to claim 8 or 9,
**characterized in that**
the peptide derivative of the general formula II is obtained by reacting a peptide sequence P₅-P'₅ with a heterobifunctional cross-linker of the general formula III 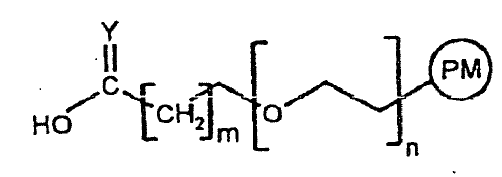 in which
Y = O or S
n = 1 to 5
m = 0 to 6
PM = a protein-binding group
in the presence of a carboxylic acid activation reagent.

11. Process according to one of the claims 8 to 10,
**characterized in that**
the carboxylic acid activation reagent is selected from N,N'-dicyclohexylcarbodiimide, N,N'-diisopropylcarbodiimide or (benzotriazol-1-yloxy)-tris (dimethylamino)phosphonium hexafluorophosphate, preferably N,N'-diisopropylcarbodiimde.

12. Pharmaceutical preparation
**characterized by**
a content of doxorubicin-peptide derivative according to one of the claims 1 to 8 as the active substance, optionally together with common pharmaceutical auxiliary agents and/or solvents.

13. Use of a doxorubicin-peptide derivative according to one of the claims 1 to 7 for the production of a pharmaceutical preparation for the treatment of cancer diseases.

14. Process for the production of a pharmaceutical preparation for the treatment of cancer diseases,
**characterized in that**
a compound according to one of the claims 1 to 7 is transferred into a therapeutically acceptable solution.

## Revendications

1. Dérivé peptidique de doxorubicine de formule générale I : 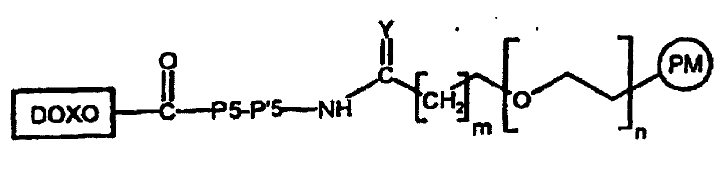 dans laquelle
Y = O ou S,
n = 1 à 5,
m = 0 à 6,
P₅-P'₅ désigne une séquence peptidique pouvant être clivée par une métalloprotéinase matricielle 2 ou 9 (MMP-2 ou MMP-9), constituée de jusqu'à dix des vingt acides aminés essentiels, et PM est un groupe se liant à une protéine, et 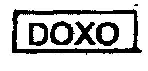 = la doxorubicine de formule 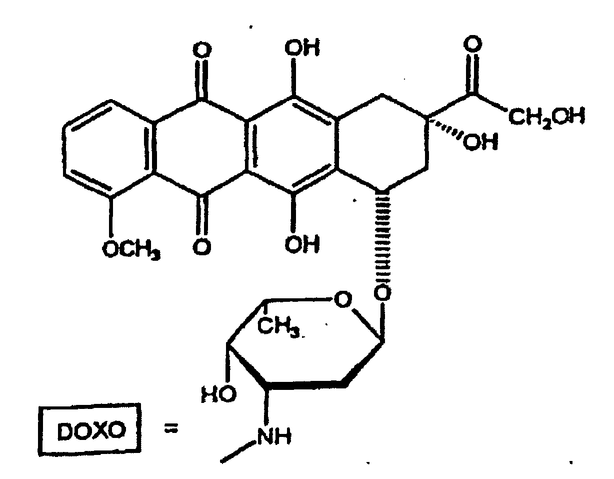 PM étant choisi parmi un groupe maléinimide, un groupe 2-dithiopyridyle, un groupe halogéno-acétamide, un groupe halogéno-acétate, un groupe disulfure, un groupe ester d'acide acrylique, un groupe ester d'acide monoalkylmaléique, un groupe amide d'acide monoalkylmaléamique, un groupe ester de N-hydroxysuccinimidyle, un groupe isothiocyanate ou un groupe aziridine qui peuvent éventuellement être substitués.

2. Dérivé peptidique de doxorubicine selon la revendication 1, dans lequel PM est un groupe maléinimide qui peut éventuellement être substitué.

3. Dérivé peptidique de doxorubicine selon la revendication 1 ou 2,
**caractérisé en ce que**
m < 2.

4. Dérivé peptidique de doxorubicine selon l'une des revendications 1 à 3, **caractérisé en ce que**
Y = O.

5. Dérivé peptidique de doxorubicine selon l'une des revendications précédentes, **caractérisé en ce que** la séquence peptidique P₅-P'₅ comprend huit acides aminés.

6. Dérivé peptidique de doxorubicine selon la revendication 5,
**caractérisé en ce que**
la séquence peptidique est Gly-Pro-Gln-Gly - Ile-Ala-Gly-Gln, Gly-Pro-Leu-Gly - Ile-Ala-Gly-Gln ou Gly-Pro-Gln-Gly - Ile-Trp-Gly-Gln.

7. Dérivé peptidique de doxorubicine selon la revendication 5,
**caractérisé en ce que**
la séquence peptidique est Gly-Pro-Leu-Gly - Ile-Ala-Gly-Gln.

8. Procédé de préparation de dérivés peptidiques de doxorubicine selon l'une des revendications précédentes,
**caractérisé en ce que**
une réaction s'effectue en présence de réactif d'activation d'acide carboxylique, avec un dérivé peptidique de formule générale II 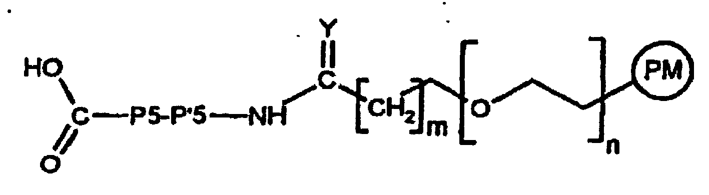 dans laquelle
Y = O ou S,
n = 1 à 5,
m = 0 à 6,
P₅-P'₅ désigne une séquence peptidique constituée de jusqu'à dix des vingt acides aminés essentiels et PM désigne un groupe se liant à une protéine.

9. Procédé selon la revendication 8,
**caractérisé en ce que**
PM est un groupe maléinimide.

10. Procédé selon la revendication 8 ou 9,
**caractérisé en ce que**
le dérivé peptidique de formule générale II est obtenu par la réaction d'une séquence peptidique P₅-P'₅ avec un agent de réticulation hétéro-bifonctionnel de formule générale III 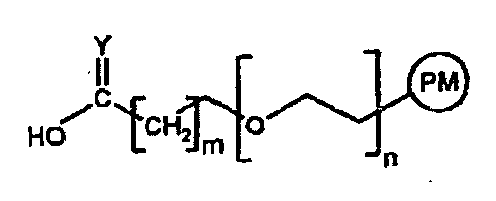 dans laquelle
Y = O ou S,
n = 1 à 5,
m = 0 à 6,
PM = un groupe se liant à une protéine,
en présence d'un réactif d'activation d'acide carboxylique.

11. Procédé selon l'une des revendications 8 à 10,
**caractérisé en ce que**
le réactif d'activation d'acide carboxylique est choisi parmi le N,N'-dicyclohexylcarbodiimide, le N,N'-diisopropylcarbodiimide ou le (benzotriazol-1-yloxy)-tris(diméthylamino)phosphonium-hexafluorophosphate, de préférence le N,N'-diisopropylcarbodiimide.

12. Médicament,
**caractérisé par**
une teneur en dérivé peptidique de doxorubicine selon l'une des revendications 1 à 8, comme principe actif, éventuellement, conjointement avec des adjuvants et/ou solvants pharmaceutiques classiques.

13. Utilisation d'un dérivé peptidique de doxorubicine selon l'une des revendications 1 à 7, pour la fabrication d'un médicament destiné au traitement de cancers.

14. Procédé de production d'un médicament pour le traitement de cancers, **caractérisé en ce que** l'on convertit un composé selon l'une des revendications 1 à 7, en une solution thérapeutiquement acceptable.
